# EUROPÄISCHE PATENTSCHRIFT

(11) **EP 1 191 965 B1**
(45) Veröffentlichungstag und Bekanntmachung des Hinweises auf die Patenterteilung: **15.01.2003**
(21) Anmeldenummer: 00952992.6
(22) Anmeldetag: 03.07.2000
(51) Int. Cl.: A61M 5/19, A61M 5/315

(54) **SPRITZVORRICHTUNG ZUR INJEKTION MINDESTENS ZWEIER FLÜSSIGER THERAPEUTIKA, INSBESONDERE INSULINE**
INJECTION DEVICE FOR INJECTING AT LEAST TWO LIQUID THERAPEUTIC AGENTS SUCH AS INSULINE
DISPOSITIF D'INJECTION SERVANT A INJECTER AU MOINS DEUX AGENTS THERAPEUTIQUES LIQUIDES, NOTAMMENT DES INSULINES

(30) Priorität: 02.07.1999 DE 19930631
(43) Veröffentlichungstag der Anmeldung: 03.04.2002
(73) Patentinhaber: Micheler, Clemens, 82319 Starnberg (DE); Ruhland, Bernd, 81377 München (DE)
(72) Erfinder: Micheler, Clemens, 82319 Starnberg (DE); Ruhland, Bernd, 81377 München (DE)
(74) Vertreter: Schmidt-Evers, Jürgen, Dipl.-Ing.
(86) Internationale Anmeldenummer: EP0006197
(87) Internationale Veröffentlichungsnummer: WO01002039

(56) Entgegenhaltungen:
- WO-A-94/11039
- US-A- 5 423 752
- US-A- 5 505 704
- US-A- 5 584 815

## Beschreibung

Die Erfindung betrifft eine Spritzvorrichtung zur Injektion mindestens zweier flüssiger Therapeutika, insbesondere Insuline, mit einem Behälter für jedes zu injizierende Therapeutikum mit einem Auslaß für jeden Behälter, mit einer gemeinsamen Spritzkanäle, in die die Auslässe aller Behälter münden, mit einer Dosiervorrichtung für jeden Behälter, mittels welcher die von dem Behälter jeweils an den zugeordneten Auslaß abzugebende Menge des Therapeutikums einstellbar ist, wobei die vorher bestimmte Menge der Therapeutika bei Betätigung einer Auslösevorrichtung von den Dosiervorrichtungen selbsttätig abgegeben wird.

Einzelspritzvorrichtungen zur Verabreichung von Insulinen sind bereits in vielfältiger Form bekannt und im Handel erhältlich. Sie werden im allgemeinen als sogenannte Pen bezeichnet.

Mit diesen Pen bzw. Insulin-Pen können im wesentlichen alle üblichen Insulintypen verabreicht werden. Üblicherweise handelt es sich dabei um langsam wirkende Insuline, die zur Basalabdeckung dienen, und um schnellwirkende Insuline.

Die bekannten Insulin-Pen sind häufig mit einer Dosiervorrichtung versehen, mit der die zu verabreichende Therapeutikamenge vorher bestimmt wird. Nachdem diese Menge eingestellt ist, wird der Insulin-Pen mit seiner Kanüle in das Unterhautgewebe eingeführt. Danach wird dann eine Auslösevorrichtung betätigt, wodurch die vorher bestimmte Therapeutikamenge selbsttätig aus einem Behälter in den Körper injiziert wird.

Nachteilig an diesem bekannten Insulin-Pen ist die Tatsache, daß mit einer Injektion nur jeweils ein Insulin-Typ pro Injektion gespritzt werden kann.

Um nun zwei verschiedene flüssige Therapeutika nacheinander oder gleichzeitig injizieren zu können, empfiehlt die EP 0 217 546 A1 eine Doppelspritze mit zwei Behältern, deren Auslässe mit einer gemeinsamen Kanüle verbunden sind. Zwischen den Auslässen und der gemeinsamen Kanüle befindet sich ein Dreiwegeventil, so daß die in jeweils einem der Behälter vorhandene Flüssigkeit alleine oder beide Flüssigkeiten gemeinsam je nach Stellung des Dreiwegeventils appliziert werden können.

Die Injektion erfolgt dabei durch Niederdrücken eines Stempels, der mit einem Kolben verbunden ist, welche wiederum die in dem jeweiligen Behälter vorhandene Flüssigkeit aus diesem quasi herausschiebt. Dabei ist pro Behälter jeweils ein Kolben vorhanden, die zwar gleichzeitig niedergedrückt werden können. Dabei kann das abzugebende Volumen jedoch nur dadurch vorherbestimmt werden, daß nur dieses Volumen in den Behälter aufgezogen wird. Zudem erfolgt die Abgabe nicht selbsttätig sondern durch manuelles Niederdrücken der Kolben.

Ferner ist aus der DE 40 21 809 A1 ein Spritzgerät zur Injektion dosierter Mengen verschiedener flüssiger Therapeutika bekannt, das über ein gemeinsames Gehäuse verfügt, in dem zwei Behälter angeordnet sind. deren Auslässe in eine gemeinsame Kanüle münden. Für jeden Behälter ist zudem ein Zähl- und Dosierwerk vorhanden, das jeweils mit einer Auslöseeinrichtung versehen ist. Bei Auslösung des Zähl- und Dosierwerkes wird auf einen verschiebbaren VerschlußlKolben im Behälter eingewirkt, so daß dieser einen bestimmten Weg im Behälter verschoben wird, wodurch die gewünschte Therapeutikamenge abgegeben wird.

Auch bei diesem bekannten Spritzgerät muß jedes Zahl- und Dosierwerk mittels einer eigenen Auslöseeinrichtung separat angesprochen werden.

Das Spritzgerät ist ferner mit zwei Auslöseeinrichlungen versehen, bei denen es sich beispielsweise um mit Betätigungsknöpfen versehenen Kolbenstangen handelt, die mit den Zähl- und Dosierwerken in Wirkverbindung stehen und anderendig mit Druckstempeln ausgerüstet sind, die auf die genannten Verschlüsse in den Behältern einwirken.

Aus der DE 39 03 315 A1 ist ferner eine Spritze mit zwei Zylindern bekannt, die mit einem flüssigen Therapeutikum gefüllt werden können und mit einem darin bewegbar gelagerten Kolben ausgestattet sind, der mit einer Kolbenstange verbunden ist. Beim Niederdrücken der Kolbenstange wird das Therapeutikum durch die mit dem Zylinder verbundene Spritzkanüle ausgebracht und beispielsweise in einen Tierkörper injiziert. Ähnliches lehrt auch die DE 297 11 075 U1.

Bei dieser bekannten Spritze können die Kolbenstangen der beiden Zylinder miteinander verbunden werden, so daß sie beim Betätigen gleichzeitig um denselben Betrag niedergedrückt werden. Unterschiedliche Therapeutikamengen pro Injektionsvorgang können mit dieser bekannten Spritze jedoch nur dann abgegeben werden, wenn die Zylinder unterschiedliche Volumina aufweisen, da ja die Kolben immer um den gleichen Weg bewegt werden.

Eine Spritzvorrichtung mit zwei Zylindern, aus denen unterschiedliche Therapeutikamengen pro Injektionsvorgang verabreicht werden können, ist aus der DE 36 09 991 A1 bekannt. Diese dort beschriebene Spritzvorrichtung besitzt zwei zusammenwirkende Kolben und Zylinder, die durch einen einzigen Vorgang einer Betätigungsvorrichtung die Injektion von zwei unterschiedlichen Flüssigkeiten in ein Tier ermöglichen. Dazu werden die Kolbenwege unterschiedlich lang eingestellt. Vor Verabreichung einer zweiten Injektion werden die Kolben dann in ihre Ausgangslage zurückbewegt.

Eine weitere Spritzvorrichtung, mit der unterschiedliche Therapeutikamengen pro Injektionsvorgang verabreicht werden können, ist aus der EP 05 38 174 A1 bekannt. Die Abgabe unterschiedlicher Therapeutikamengen wird dabei dadurch erreicht, daß bei dieser bekannten Vorrichtung Einzelspritzvorrichtungen unterschiedlicher Volumina eingesetzt werden.

Eine Spritzvorrichtung der eingangs beschriebenen Art ist in der WO 94/11039 beschrieben. Bei dieser bekannten Spritzvorrichtung ist eine gemeinsame Auslösevorrichtung für beide Dosiervorrichtungen vorgesehen.

Aufgabe der vorliegenden Erfindung ist es, die zuletzt erwähnte bekannte Spritzvorrichtung konstruktiv zu vereinfachen.

Gelöst wird diese Aufgabe durch eine Spritzvorrichtung die dadurch gekennzeichnet ist, daß es sich bei den Dosiervorrichtungen um solche von handelsüblichen Einzelspritzvorrichtungen, insbesondere für Insuline, handelt, die jeweils mit einer separaten Auslösevorrichtung vorsehen sind, und daß die Auslösevorrichtungen gleichzeitig mittels einer gemeinsamen Betätigungsvorrichtung betätigbar sind, welche einerseits mit den separaten Auslösevorrichtungen in dem gemeinsamen Gehäuse in Wirkverbindung steht und andererseits aus dem gemeinsamen Gehäuse herausragt.

Die pro Behälter abzugebende Therapeutikamenge kann dabei unabhängig von dem anderen oder den anderen Behältern frei gewählt werden. Mit anderen Worten, die pro Behälter abzugebende Therapeutikamenge ist unabhängig von den für die anderen Behälter eingestellten, abzugebenden Therapeutikamengen.

Zudem erfolgt die Abgabe selbsttätig. Dies bedeutet, daß bei der Betätigung der Auslösevorrichtung, mit der die Dosiervorrichtungen versehen sind, durch die Betätigungsvorrichtung die vorher eingestellte Therapeutikamenge abgegeben wird, ohne daß dies, nachdem die gewünschte Therapeutikamenge eingestellt wurde, von der handhabenden Person noch zu beeinflussen ist. Beispielsweise kann dies dadurch erfolgen, daß die Dosiervorrichtung mit einer Feder zusammenwirkt. die eine in dem Behälter befindliche Kolbenstange und den damit verbundenen Kolben oder ähnliches um einen gewünschten Betrag bzw. Weg verschiebt, so daß die entsprechende Flüssigkeitsmenge und somit auch Therapeutikamenge abgegeben wird. Die Art, wie die Dosierung und somit das Abgeben des flüssigen Therapeutikums aus dem Behälter erfolgt, kann dabei beliebig sein. Beispielsweise ist es auch möglich, elektrische Dosierpumpen oder ähnliches zur Anwendung zu bringen.

Durch das Betätigen der Betätigungsvorrichtung wird somit nicht die Kraft aufgewandt, die erforderlich ist, um die Therapeutika aus ihrem Behältnis abzugeben. Werden die Therapeutika beispielsweise mit Hilfe eines Kolbens und einer Kolbenstange aus einem Behältnis herausgedrückt, dann wird durch Betätigen der Betätigungsvorrichtung nicht etwa die Kolbenstange und somit auch der Kolben in das Behältnis hineingedrückt. Vielmehr wirkt die Betätigungsvorrichtung lediglich auf die Auslösevorrichtung der Dosiervorrichtung ein, die dann selbsttätig dafür Sorge trägt, daß die gewünschte Therapeutikamenge abgegeben wird.

Eines der wesentlichen Merkmale der Erfindung besteht somit darin, daß die Betätigungsvorrichtung alle Auslösevorrichtungen der Dosiervorrichtungen gleichzeitig und gemeinsam betätigt. Bei den Auslösevorrichtungen kann es sich hier beispielsweise um Knöpfe oder Hebel handeln, die niedergedrückt werden müssen, damit die Dosiervorrichtung im Zusammenwirken mit dem Behälter die gewünschte Therapeutikamenge abgibt. In diesem Fall wirkt die Betätigungsvorrichtung gleichzeitig auf alle derartigen Knöpfe und Hebel, so daß der gewünschte Effekt erzielt wird.

Nach einer bevorzugten Ausführungsform werden die Therapeutikamenge aus dem dazugehörigen Behälter mittels eines darin beweglichen Kolbens, der mit einer Kolbenstange ausgestattet sein kann, ausgestoßen und somit abgegeben. In diesem Falle handelt es sich bei dem dazugehörigen Behälter meistens um ein Glasröhrchen; derartige Ausgestaltungen sind bekannt. Der Kolben verbleibt dann nach Abgabe der vorher festgelegten bestimmten Therapeutikamenge an seinem letzten Endpunkt und wird nicht mehr auf seinen Ausgangspunkt zurückgeführt. Mit anderen Worten, der Kolben bleibt stehen und wird in Richtung des Auslaßes weiterbewegt, wenn die nächste Therapeutikamenge abgegeben werden soll. Der Kolben bewegt sich somit immer von seiner Ausgangsposition weiter weg und wandert nicht wieder dahin zurück. Auf diese Weise wird in dem Behälter kein Vakuum erzeugt, so daß auch keine Verschmutzungen oder ähnliches in den Behälter oder die Kanülen etc. gelangen können.

Nach einer weiterhin bevorzugten Ausführungsform sind die Dosiervorrichtungen derart ausgestaltet, daß die Therapeutikamenge vor jeder Abgabe der Therapeutika einstellbar ist. Die Einstellung der mit einer Gabe abzugebenden Therapeutikamenge gilt in diesem Fall somit nur für einen Betätigungsvorgang bzw. für eine einzige Verabreichung der gewünschten Therapeutikamengen. Für die nächste Abgabe muß dann die dabei abzugebende nächste Therapeutikamenge erneut eingestellt werden.

Dies kann so oft wiederholt werden, bis die Behälter entleert sind. Danach können dann die Behälter entweder erneut gefüllt oder ausgetauscht werden. Dafür ist es dann natürlich erforderlich, daß der bewegliche Kolben in seine Ausgangslage bzw. Ausgangsposition zurückbefördert wird.

Die erfindungsgemäßen Dosiervorrichtungen von handelsüblichen Einzelspritzvorrichtungen können beispielsweise solche von handelsüblichen Insulin-Pen sein, die nur noch in das gemeinsame Gehäuse zu inkorporieren sind. Die vorstehend erwähnten Dosiervorrichtungen von handelsüblichen Einzelspritzvorrichtungen sind, ein Kennzeichnendes Teil der Erfindung. Ferner müssen die Kanülen, die aus diesen handelsüblichen Insulin-Pen austreten, vereinigt werden, so daß die aus den Kanülen austretenden Flüssigkeiten in die gemeinsame Spritzkanüle gelangen und von dort in den Körper injiziert werden. Das Gehäuse kann die handelsüblichen Insulin-Pen zumindest teilweise umgeben. Allerdings muß sichergestellt werden, daß die Dosiervorrichtung zum Einstellen der abzugebenden Dosis zugänglich bleiben. Im einfachsten Fall kann ein derartiges Gehäuse somit aus Klammern oder ähnlichem bestehen.

Bei der Betätigungsvorrichtung handelt es sich vorzugsweise um eine Stange, die an einem Ende mit den Auslösevorrichtungen der handelsüblichen Einzelspritzvorrichtungen in Wirkverbindung steht und am anderen Ende aus dem Gehäuse hinausragt hinausragt, so daß diese Betätigungsvorrichtung gut zugänglich ist.

Die Behälter, die bei den handelsüblichen Insulin-Pen in diese integriert sind und in eine Umhüllung eingesetzt sind, können in diesem Fall separat gehaltert werden, indem sie beispielsweise in das Gehäuse der erfindungsgemäßen Spritzvorrichtung derart integriert werden, daß die Dosiervorrichtungen die flüssigen Therapeutika auf die gleiche Weise wie bei den bekannten Insulin-Pen aus den Behältern abgeben bzw. hinausdrücken können. Dies kann auf mechanischem oder auch auf elektrischem oder anderem Wege geschehen. Die Dosiervorrichtungen üblicher Insulin-Pen sind mit einem Stempel ausgestattet, der auf einen im Behälter beweglich gelagerten Verschluß bzw. Kolben einwirkt. Bei Bewegung des Stempels, der auch als Kolbenstange bezeichnet werden kann, wird der Verschluß bzw. der Kolben in dem Behälter bewegt, so daß die gewünschte Therapeutikamenge am anderen Ende des Behälters ausgetrieben wird.

Bei den Behältern für die erfindungsgemäße Spritzvorrichtung handelt es sich vorzugsweise um auswechselbare Behälter, die auch in üblichen Insulin-Pen Anwendung finden. Diese stellen normalerweise Glasröhrchen dar, deren Innenraum auf einer Seite durch einen verschiebbaren Kolben bzw. Verschluß aus Gummi abgedichtet ist. Am andere Ende verjüngt sich das Röhrchen und ist dort mit einer Membran ausgestattet, in die eine Kanüle oder ein Auslauf hineingestochen werden kann.

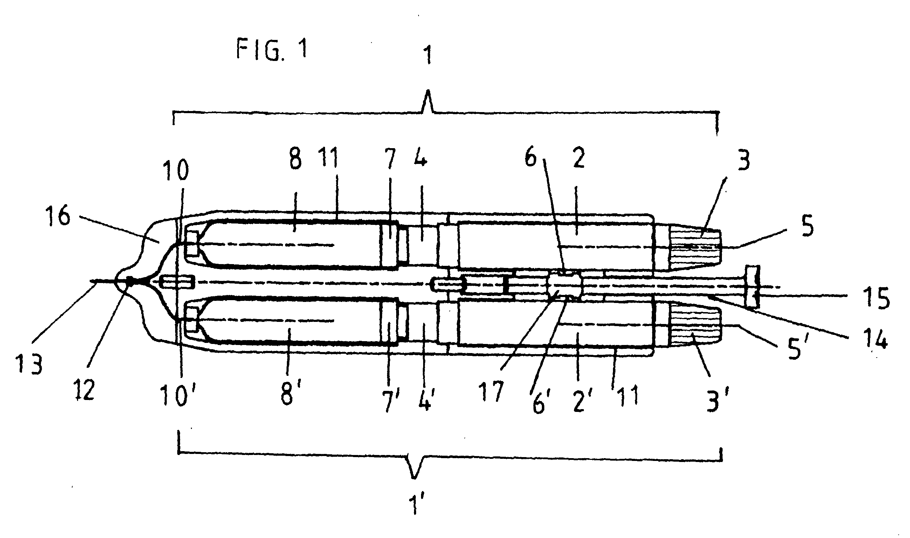

Die Erfindung wird im folgenden anhand der Fig. 1 näher erläutert, die eine bevorzugte erfindungsgemäße Spritzvorrichtung in skizzenhafter Weise darstellt. Es handelt sich dabei um eine Art Aufsicht auf eine zwei Spritzeinheiten 1,1' aufweisende Spritzvorrichtung, wobei die beiden Spritzeinheiten 1,1' in einem gemeinsamen Gehäuse 11 angeordnet sind und auch zeichnerisch angedeutet sind.

Die in der Fig. 1 gezeigte, bevorzuge Ausführungsform der erfindungsgemäßen Spritzvorrichtung besitzt somit zwei Spritzenabschnitte 1,1' mit jeweils einer erfindungsgemäßen Dosiervorrichtung 2,2', bei denen es sich um Dosiervorrichtungen eines handelsüblichen Insulin-Pen handelt. Diese Dosiervorrichtungen 2,2' sind stiftartig ausgebildet, weisen an einem Ende eine um die Längsachse drehbare Einstellkappe 3,3' auf und sind am anderen Ende mit einem Stempel 4,4' versehen. Die abzugebende Therapeutikamenge wird dadurch eingestellt, daß die Einstellkappen 3,3' um die Längsachse 5,5' gedreht wird und in der Endstellung arretieren. Je größer der Winkelbetrag der Drehung ist, desto größer ist auch die abgegebene Therapeutikamenge bzw. desto weiter wird der Stempel 4,4' bei Auslösung aus der Dosiervorrichtung 2,2' herausgedrückt.

Die Dosiervorrichtungen 2,2' sind zu diesem Zweck mit nicht gezeigten Federn ausgestattet. Beim Betätigen der Auslösevorrichtungen 6,6', bei denen es sich im gezeigten Falle um einen in Richtung der Längsachse 5,5' verschiebbar geführte Knöpfe handelt, werden die Feder quasi freigegeben und drücken die Stempel 4,4' den gewünschten Betrag hinaus (bezogen auf die Zeichnung der Fig. 1 nach links).

Die Stempel 4,4' drücken dazu auf einen Kolben bzw. Verschluß 7,7', der beweglich in dem Behälter 8,8' geführt ist. Bei den Behältern 8,8' handelt es sich um übliche, standardisierte Glasampullen für Insulin-Pen. Auf der den Dosiervorrichtungen 2,2' abgewandten Seite besitzen die Behälter 8,8' eine Membran oder einen üblichen Gummistopfen 9,9', durch die eine Auslaßkanüle 10,10' hindurchgeführt bzw. hindurchgestochen werden kann. Die Behälter 8,8' sind auswechselbar in dem Gehäuse 11 gehaltert bzw. gelagert. Auch die Dosiervorrichtungen 2,2' sind in diesem Gehäuse 10,10' gehaltert.

Die beiden Auslaßkanülen 10,10' münden beide in eine Kammer 12, die wiederum in eine gemeinsame Spritzkanüle 13 übergeht, aus deren freiem Ende bzw. aus deren Spitze die abzugebende Flüssigkeit austritt.

Die erfindungsgemäße Spritzvorrichtung ist mit nur einer Betätigungsvorrichtung 14 versehen, die mit beiden Auslösevorrichtungen 6,6' in Wirkverbindung steht. Bei der gezeigten Ausführungsform ist dies dadurch verwirklicht, daß die beiden Auslösevorrichtungen 6,6' der Dosiervorrichtungen 2,2' einander gegenüberliegend angeordnet sind. Bei der Betätigungsvorrichtung 14 handelt es sich um eine Stange, deren eines Ende 17 zwischen den beiden Auslösevorrichtungen 6,6' zu liegen kommt und damit formschlüssig verbunden ist. So können beispielsweise die Auslösevorrichtungen 6,6' als eine Art Zahnstange ausgebildet sein, in die dieses Ende 17 eingreift, welches ebenfalls als eine Zahnstange ausgebildet ist, jedoch als doppelseitige Zahnstange, um in die beiden Auslösevorrichtungen formschlüssig einzugreifen.

Am anderen Ende der Betätigungsvorrichtung 14 befindet sich ein Betätigungsknopf 15.

Das Gehäuse 11 ist im wesentlichen aus zwei Halbschalen aufgebaut, deren Trennungsebene in etwa parallel zur Papierebene der Fig. 1 liegt. An einem Ende des Gehäuses 11 ragen die Einstellkappen 3,3' sowie die Betätigungsvorrichtung 14 heraus. Am andere Ende des Gehäuses 11 ist ein deckelartiger Aufsatz 16 vorgesehen, der die Spritzkanüle 13 haltert und die Kammer 12 aufweist. Zudem befinden sich dort die Auslaßkanäle 10,10', die über die Grenzfläche 17 hinausragen, und zwar so weit, daß sie sich im zusammengebauten Zustand durch die Membrane bzw. Gummistopfen 9,9' bis in den Innenraum der Behälter 8,8' erstrekken.

Die beiden Schalen bzw. Hälften des Gehäuses 11 können noch einmal jeweils in zwei Teile unterteilt sein, die in etwa im Bereich der Stempel 4,4' aneinander stoßen.

Die Ausgestaltung des Gehäuses 11 kann dabei beliebig sein. Die Teile können auf beliebige Art und Weise miteinander verbunden werden, beispielsweise durch Stecken. Ferner können ggf. Verbindungsschrauben oder Klammern oder ähnliches zum Einsatz gebracht werden. Das Gehäuse 11 bzw. seine Teile einschließlich des deckelartigen Aufsatzes 16 können aus einem geeigneten Kunststoff oder einem anderen geeigneten Material gefertigt sein. Zur Aufnahme bzw. Halterung von den Behältern 8,8' und den Dosiervorrichtungen 2,2' können das Gehäuse 11 oder die Gehäuseteile Ausnehmungen oder ähnliches aufweisen, in die Behälter/Dosiervorrichtungen zu liegen kommen. Diese Ausnehmungen sind beispielsweise formkomplementär dazu ausgestaltet.

Zur Injektion der gewünschten Therapeutikamenge dreht die handhabende Person die Einstellkappen 3,3' um den gewünschten Winkelbetrag. Die abzugebende Menge pro Spritzeinrichtung (Dosiervorrichtung 2 plus Behälter 8) ist dabei frei und unabhängig voneinander wählbar. Danach sticht die Person die Spritzkanüle 13 in das gewünschte Gewebe und drückt im Anschluß daran auf den Druckknopf 15 der Betätigungsvorrichtung 14. Dabei werden die Auslaßvorrichtungen 6 bewegt; die Dosiervorrichtungen 2,2' geben die voreingestellten Therapeutikamengen von sich aus ab, indem die federbeaufschlagten Stempel 4,4' um einen der abzugebenden Therapeutikamenge entsprechenden Weg in die Behälter 8,8' hineingedrückt werden.

Die Kolben bzw. Verschlüsse 7,7' verbleiben dann an demjenigen Punkt, den sie nach Abgabe der gesamten voreingestellten Therapeutikamenge erreicht haben und werden erst bei der nächsten gewünschten Abgabe des Therapeutikums erneut um den gewünschten Betrag in Richtung der Auslässe 10,10' verschoben.

Auf diese Weise werden die in den Behältern 8,8' vorhandenen Flüssigkeiten durch die Spritzkanüle 13 in der gewünschten Menge gemeinsam und gleichzeitig an den gewünschten Ort injiziert.

## Patentansprüche

1. Spritzvorrichtung zur Injektion mindestens zweier flüssiger Therapeutika, insbesondere Insuline, mit folgenden Merkmalen:
mit einem gemeinsamen Gehäuse (11),
mit einem Behälter (8, 8') für das zu injizierende Therapeutikum,
mit einem Auslaß (10, 10') für jeden Behälter (8, 8'),
mit einer gemeinsamen Spritzkanüle (13), in die die Auslässe (10, 10') aller Behälter (8, 8') münden, und
mit einer Dosiervorrichtung (2, 2') für jeden Behälter (8, 8'), mittels welcher die von dem Behälter (8, 8') jeweils an den zugeordneten Auslaß (10, 10') abzugebende Menge des Therapeutikums einstellbar ist,
wobei die vorher bestimmte Menge der Therapeutika bei Betätigung einer Auslösevorrichtung (6, 6') von den Dosiervorrichtungen (2, 2') selbsttätig abgegeben wird,
**dadurch gekennzeichnet,**
**daß** es sich bei den Dosiervorrichtungen (2, 2') um solche von handelsüblichen Einzelspritzvorrichtungen, insbesondere für Thsuline, handelt, die jeweils mit einer separaten Auslösevorrichtung (6,6') versehen sind, und
**daß** die Auslösevorrichtungen (6, 6') gleichzeitig mittels einer gemeinsamen Betätigungsvorrichtung (14) betätigbar sind, welche einerseits mit den separaten Auslösevorrichtungen (6, 6') in dem gemeinsamen Gehäuse (11) in Wirkverbindung steht und andererseits aus dem gemeinsamen Gehäuse (11) herausragt.

2. Spritzvorrichtung nach Anspruch 1,
**dadurch gekennzeichnet,**
**daß** die Therapeutikamenge aus den Behältern (8, 8') mittels jeweils eines darin beweglichen Kolbens (7, 7') ausgestoßen und somit abgegeben wird und daß die Kolben (7, 7') nach Abgabe der vorher eingestellten Therapeutikamengen am letzten Endpunkt verbleiben.

3. Spritzvorrichtung nach Anspruch 1 oder 2,
**dadurch gekennzeichnet,**
**daß** die Dosiervorrichtungen (2, 2') derart ausgestaltet sind, daß die jeweilige Therapeutikummenge vor jeder Abgabe der Therapeutika einstellbar ist.

4. Spritzvorrichtung nach Anspruch 1,
**dadurch gekennzeichnet,**
**daß** die Betätigungsvorrichtung (14) eine Stange ist.

5. Spritzvorrichtung nach einem der vorhergehenden Ansprüche,
**dadurch gekennzeichnet,**
**daß** es sich bei den Dosiervorrichtungen von handelsüblichen Einzelspritzvorrichtungen (1, 1') um solche von handelsüblichen Insulin-Pen handelt.

6. Spritzvorrichtung nach einem der vorhergehenden Ansprüche,
**dadurch gekennzeichnet,**
**daß** die Behälter (8, 8') auswechselbar sind.

7. Spritzvorrichtung nach einem der vorhergehenden Ansprüche,
**dadurch gekennzeichnet,**
**daß** die Behälter (8, 8') übliche Standardbehälter sind, die in üblichen Insulin-Pen eingesetzt werden.

8. Spritzvorrichtung nach einem der vorhergehenden Ansprüche,
**dadurch gekennzeichnet,**
**daß** die Auslässe (10, 10') in eine Kammer (12) münden, die mit der gemeinsamen Spritzkanüle (13) verbunden ist.

## Claims

1. Injection device for the injection of at least two liquid therapeutic agents, in particular insulins, having the following features:
having a common housing (11),
having a container (8, 8') for each therapeutic agent to be injected,
having an outlet (10, 10') for each container (8, 8'),
having a common injection channel (13) into which the outlets (10, 10') of all containers (8, 8') open, and
having a metering device (2, 2') for each container (8, 8') by means of which the quantity of the therapeutic agent to be respectively delivered from the container (8, 8') to the associated outlet (10, 10') can be set,
the previously determined quantities of the therapeutic agents being automatically delivered by the metering devices (2, 2') upon actuation of a trigger device (6, 6'),
**characterised in that**,
the metering devices (2, 2') are those of commercially conventional individual injection devices, in particular for insulins, which are each provided with a separate trigger device (6, 6'), and
the trigger devices (6, 6') can be simultaneously actuated by means of a common actuation device (14) which on the one hand stands in working connection with the separate trigger devices (6, 6') in the common housing (11) and on the other hand projects out of the common housing (11).

2. Injection device according to claim 1,
**characterised in that**,
the quantities of therapeutic agents are ejected, and thus delivered, out of the containers (8, 8') by means of respective pistons (7, 7') moveable therein, and **in that** after delivery of the previously set quantities of therapeutic agents the pistons (7, 7') remain at the last end point.

3. Injection device according to claim 1 or 2,
**characterised in that**,
the metering devices (2, 2') are so configured that the respective quantity of therapeutic agent can be set before each delivery of the therapeutic agents.

4. Injection device according to claim 1,
**characterised in that**,
the actuating device (14) is a rod.

5. Injection device according to any preceding claim,
**characterised in that**,
the metering devices of commercially conventional individual injection devices (1, 1') are those of commercially conventional insulin pens.

6. Injection device according to any preceding claim,
**characterised in that**,
the containers (8, 8') are exchangeable.

7. Injection device according to any preceding claim,
**characterised in that**,
the containers (8, 8') are conventional standard containers as employed in conventional insulin pens.

8. Injection device according to any preceding claim,
**characterised in that**,
the outlets (10, 10') open into a chamber (12) which is connected with the common injection channel (13).

## Revendications

1. Dispositif d'injection destiné à injecter au moins deux agents thérapeutiques liquides, notamment de l'insuline, comportant les particularités suivantes :
un logement commun (11)
un récipient (8,8') pour chaque agent thérapeutique à injecter,
une sortie (10,10') pour chaque récipient (8,8'),
une canule d'injection commune (13), dans laquelle aboutissent les sorties (10,10') de tous les récipients (8,8'), et
un dispositif de dosage (2,2') pour chaque récipient (8,8'), lequel permet d'ajuster la quantité de l'agent thérapeutique à administrer depuis le récipient (8,8') respectivement à la sortie affectée (10,10'),
moyennant quoi la quantité préalablement déterminée des agents thérapeutiques est administrée automatiquement lors de l'actionnement d'un dispositif de déclenchement (6,6') par les dispositifs de dosage (2,2'),
**caractérisé en ce que**,
les dispositifs de dosage (2,2'), sont des dispositifs d'injection individuels tels que disponibles dans le commerce, notamment pour l'insuline, qui sont respectivement équipés de dispositifs de déclenchement séparés (6,6'), et
**en ce que** les dispositifs de déclenchement (6,6') peuvent être actionnés simultanément au moyen d'un dispositif d'actionnement commun (14) qui, d'une part, est en communication opérante avec les dispositifs de déclenchement séparés (6,6') dans le logement commun (11) et qui, d'autre part, fait saillie hors du logement commun (11).

2. Dispositif d'injection selon la revendication 1,
**caractérisé en ce que**,
la quantité d'agent thérapeutique est expulsée du récipient (8,8') au moyen respectivement d'un piston mobile dans celui-ci (7,7') pour être ainsi délivrée et **en ce qu'**après délivrance de la quantité d'agent thérapeutique préalablement ajustée, les pistons demeurent sur la dernière position d'extrémité.

3. Dispositif d'injection selon la revendication 1 ou 2,
**caractérisé en ce que**,
les dispositifs de dosage (2,2') sont conçus de telle sorte que les quantités respectives d'agent thérapeutique peuvent être ajustées avant chaque délivrance des agents thérapeutiques.

4. Dispositif d'injection selon la revendication 1,
**caractérisé en ce que**,
le dispositif d'actionnement (14) est une tige.

5. Dispositif d'injection selon l'une des revendications précédentes,
**caractérisé en ce que**,
les systèmes de dosage des dispositifs d'injection individuels du commerce (1,1'), sont des pompes à insuline disponibles dans le commerce.

6. Dispositif d'injection selon l'une des revendications précédentes,
**caractérisé en ce que**;
les récipients (8,8') sont interchangeables.

7. Dispositif d'injection selon l'une des revendications précédentes,
**caractérisé en ce que**,
les récipients (8,8') sont des récipients usuels normalisés qui sont insérables dans la pompe à insuline usuelle.

8. Dispositif d'injection selon l'une des revendications précédentes,
**caractérisé en ce que** les sorties (10,10') débouchent dans une chambre qui est raccordée à la canule d'injection commune (13).
